# FASCICULE DE BREVET EUROPEEN

(11) **EP 4 064 844 B1**
(45) Date de publication et mention de la délivrance du brevet: **25.02.2026**
(21) Numéro de dépôt: 20824301.4
(22) Date de dépôt: 26.11.2020
(51) Int. Cl.: A23B 2/729, A23B 2/783, A21D 8/04, A21D 10/00

(54) **PROCÉDÉ DE CONSERVATION DES PRODUITS CUITS DE BOULANGERIE**
VERFAHREN ZUR KONSERVIERUNG VON GEBACKENEN BACKWAREN
METHOD FOR PRESERVING COOKED BAKERY PRODUCTS

(30) Priorité: 26.11.2019 FR 1913214
(43) Date de publication de la demande: 05.10.2022
(73) Titulaire: Lesaffre et Compagnie, 75001 Paris (FR)
(72) Inventeur: BRYCKAERT, Emilie, 59118 WAMBRECHIES (FR); DELCHAMBRE, Florence, 59890 QUESNOY-SUR-DEULE (FR); SOUPIRON, Laurent, 59272 DON (FR)
(74) Mandataire: Plasseraud IP
(86) Numéro de dépôt international: PCT/FR2020/052178
(87) Numéro de publication internationale: WO 2021/105616

(56) Documents cités:
- WO-A1-2014/152022
- WO-A1-2016/004182
- WO-A2-2009/097333
- US-A1- 2003 161 911

## Description

L'invention se rapporte à un procédé de préparation de produit cuit de boulangerie permettant une longue conservation sans apparition de moisissures.

Le but de l'invention, est de proposer aux professionnels et aux consommateurs, des produits cuits de boulangerie résistants aux moisissures et qui peuvent se conserver pendant plusieurs semaines sans que leurs aspects, goûts et arômes soient altérés et sans ajout de produits non naturels.

Les produits de panification ou produits cuits de boulangerie, surtout s'ils sont peu ou non sucrés et s'ils sont tranchés et vendus en tranches sont soumis au développement de moisissures lors de leur conservation. Les moisissures apparaissent dès les premiers jours de conservation.

Les moisissures sont dues à des contaminants ubiquitaires de l'atmosphère d'un fournil, qui viennent se déposer sur les tranches de pain. Certaines moisissures sont productrices de toxines dont la consommation est dommageable pour la santé. En conséquence, il est souvent indispensable d'ajouter dans la composition des pains, et plus particulièrement les pains tranchés longue conservation, lors de leur fabrication, des anti-moisissures ou antifongiques comme l'acide acétique ou ses sels, l'acide propionique ou ses sels, l'acide sorbique ou ses sels. L'acide benzoïque et ses sels, les esters de l'acide p-hydroxybenzoïque ou de ses sels encore appelés PHB ou parabens sont d'autres conservateurs utilisés classiquement dans le domaine des produits alimentaires. De manière générale, les inhibiteurs de moisissures sont des acides faibles et/ou des sels d'acide faible et l'expression inhibiteur de moisissures désigne l'ensemble de ces produits, y compris tous les acides faibles utilisés aussi comme agent acidifiant.

Ces antifongiques ou anti-moisissures sont des produits conservateurs non naturels. Or, de nos jours, de plus en plus de consommateurs, sont à la recherche de produits sans conservateurs. La tendance du « clean label » est très forte et les produits conservateurs non naturels doivent être évités.

L'alcool peut également être appliqué en pulvérisation sur produit cuit ou injecté dans le produit cuit pour ses propriétés antifongiques. Cependant, les produits résultants de cette application d'alcool sont inadaptés aux enfants et à une partie de la population qui n'absorbe pas d'alcool.

Ces antifongiques ont une action inhibitrice des levures de panification plus ou moins importante. En pratique, c'est le propionate de calcium qui est le plus utilisé en panification. Il a une action modérée mais certaine d'inhibition des levures de panification. Son emploi conduit à des apprêts, appelés proof times, plus longs et à des pains ayant des mies plus ouvertes.

Le pain dit « pain de mie » en France ou « sandwich bread » aux Etats-Unis, contenant de la matière grasse et du sucre, tranché, est destiné à être conservé longtemps et pour ce faire, l'ajout d'anti-moisissures est nécessaire. Il en est de même pour les brioches, pains au lait, buns ou autres produits de panification longue conservation.

De manière générale, le sandwich bread ou les buns sont préparés par un procédé indirect de panification.

Un tel procédé indirect de panification est un procédé qui comporte une première étape de pré-fermentation mettant en œuvre une partie de la farine, de l'eau et de la levure de la recette totale. Cette première étape de pré-fermentation est appelée dans la terminologie de la boulangerie française un levain levure ou une poolish ou encore un sponge selon la consistance de la pâte. Ces procédés indirects avec une pré-fermentation avec de la levure de panification sont peu utilisés en France mais représentent l'essentiel des procédés utilisés aux Etats-Unis d'Amérique (USA). L'étape correspondant à la formation d'un levain levure, qui est un pré-ferment sous forme d'une masse pâteuse en fermentation alcoolique par de la levure, est plus connue sous le terme américain sponge.

Le sponge désigne la première étape de pré-fermentation avec en général au moins 40% de la farine mise en œuvre dans la recette totale, une majorité de la levure et de l'eau mises en œuvre dans la recette totale. Le terme dough qui signifie pâte en français, correspond à l'ensemble des étapes de fabrication d'un pain (l'étape finale conduisant à la cuisson du pain).

L'intérêt de l'étape de pré-fermentation est de permettre à la levure de produire un ensemble de métabolites qui vont améliorer le goût et la texture du pain obtenu. Cela est en général d'autant plus marqué que le sponge contient une plus grande proportion de la farine de la recette totale.

Comme cette étape de pré-fermentation est destinée à exprimer au maximum le potentiel fermentaire de la levure et comme il est admis que pendant les 45 premières minutes de la fermentation panaire la levure est très sensible à son environnement (page 629, volume II, Baking Science and Technology, E.J. Pyler, voir aussi formule type page 591), la règle est généralement d'ajouter le ou les inhibiteurs de moisissures lors de la deuxième étape de la panification, à savoir l'étape du dough. Cette deuxième et dernière étape comprend les étapes classiques de fabrication du pain, à savoir notamment un pétrissage avec tous les ingrédients (tels que farine, eau, sel, levure, poolish etc.), une division de la pâte, un façonnage et une mise en moule, un apprêt ou proof-time en étuves et la cuisson. Ainsi, de façon générale, le ou les inhibiteurs de moisissure sont ajoutés lors de l'étape définie ci-dessus comme l'étape du dough.

Le problème de l'utilisation des inhibiteurs de moisissures dans les pains et les autres produits levés à la levure est qu'ils inhibent les cellules de levures en même temps que les moisissures non voulues. Ceci résulte en une réduction de l'activité de la levure et de la production de gaz.

Différentes méthodes ont été employées pour surmonter ces problèmes. Comme mentionné ci-dessus, la plus commune est de toujours ajouter l'inhibiteur à l'étape du dough et jamais lors de l'étape du sponge ou du ferment liquide, car c'est lors de cette première étape que l'inhibiteur a le plus d'opportunité d'avoir une action préjudiciable. Mais cette méthode a aussi montré ses limites.

L'utilisation de ces inhibiteurs non naturels est inadaptée à la tendance actuelle du « clean label ». Par ailleurs, l'utilisation d'alcool, même si elle s'avère efficace pour son activité anti-moisissure n'est pas adaptée à l'ensemble de la population. Le vinaigre est l'une des seules alternatives « clean label » mais présente une efficacité moindre.

Parmi les autres moyens de lutter contre les moisissures on peut citer ceux décrits dans WO 2009/097333 et dans US 2003/0161911.

La méthode de conservation proposée par WO 2009/097333 consiste en l'application sur les produits à conserver d'une composition comprenant de la levure vivante. L'application peut être opérée par tout moyen juste avant l'emballage des produits à protéger. Cependant avec cette méthode la conservation est assurée au maximum pendant 21 jours.

US 2003/0161911 décrit des souches de levures destinées à la panification de pâtes comprenant au moins 5% de sucres. Il décrit aussi les procédés de panification correspondants qui permettent l'obtention de pains ayant un taux d'éthanol inférieur à 1,5% (w/w) qui permet une résistance suffisante à l'apparition des moisissures.

Les solutions décrites précédemment ne sont pas entièrement satisfaisantes dans la mesure où elles ne permettent pas de répondre aux besoins du marché en termes de temps de conservation des pains, notamment les pains tranchés et peuvent compliquer le travail du boulanger. Les professionnels et les consommateurs sont de plus en plus exigeants et demandent la mise au point de pains tranchés qui restent en bon état de conservation pendant plusieurs semaines tout en gardant toutes leurs propriétés et qualités organoleptiques et sans ajout de produits non naturels.

Après de nombreux essais, les inventeurs ont constaté que l'apparition des moisissures sur un pain tranché, tel que le pain de mie français ou encore le pain américain, est considérablement ralentie lorsque ledit pain est préparé selon un procédé comprenant la cuisson d'une pâte obtenue par une méthode de panification de type sponge and dough et lorsque ces pains sont traités par l'application en surface d'une composition comprenant un levain vivant et/ou une levure vivante.

### Résumé de l'invention

Ainsi, il est proposé un procédé de préparation de produit cuit de boulangerie résistant aux moisissures comprenant la cuisson d'une pâte obtenue par une méthode de panification de type sponge and dough mettant en œuvre une poolish contenant de 20 à 55 g/L d'éthanol, de préférence de 35 à 50 g/L d'éthanol et l'application sur le pain cuit d'une composition comprenant un levain vivant et/ou une levure vivante.
Selon un mode de réalisation avantageux de l'invention la poolish est incorporée avec l'ensemble des ingrédients de panification lors de l'étape du pétrissage.

Selon un autre aspect, il est proposé l'utilisation d'une poolish contenant de 20 à 55 g/L d'éthanol, de préférence de 35 à 50 g/L d'éthanol, conjointement avec l'application d'une composition de levain vivant et/ou de levure vivante à la surface d'un produit de boulangerie cuit, pour ralentir l'apparition de moisissures sur le produit de boulangerie cuit, notamment tranché.

L'invention est définie par les revendications 1-14 annexées.

### Exposé de l'invention

L'invention porte sur un procédé de préparation de produit cuit de boulangerie résistant aux moisissures comprenant la cuisson d'une pâte obtenue par une méthode de panification de type sponge and dough mettant en œuvre une poolish contenant de 20 à 55 g/L d'éthanol, de préférence de 35 à 50 g/L d'éthanol et l'application sur le pain cuit d'une composition comprenant un levain vivant et/ou une levure vivante.

Ce procédé est particulièrement utile pour les produits cuits de boulangerie incorporant du sucre et des matières grasses, tels que le pain de mie ou les pains américains. Ces produits cuits de boulangerie sont dans la majorité des cas proposés aux clients sous forme de produit tranché mis en sachet. Il est important que leur conservation soit assurée pendant plusieurs semaines.

Au sens de la présente invention, on entend par « conservation » l'absence de développement de moisissures en surface du produit cuit de boulangerie et le maintien des qualités organoleptiques du produit cuit de boulangerie pendant au moins 21 jours, de préférence au moins 28 jours à la température ambiante.

La mise en œuvre d'un procédé indirect avec une poolish en combinaison avec l'application après cuisson d'un levain vivant et/ou d'une levure vivante permet d'augmenter la conservation du produit cuit de boulangerie en retardant la formation de moisissures.

Dans ce qui suit, on utilisera indifféremment le terme pré-ferment ou poolish ou sponge.

Même si la teneur en éthanol dans la poolish mise en œuvre dans le procédé selon l'invention peut être élevée, elle n'a pas d'incidence sur les qualités organoleptiques du produit cuit de boulangerie finalement obtenu. Une grande quantité, voire la majeure partie de l'éthanol s'évapore lors de la cuisson. La teneur en éthanol après la cuisson est de l'ordre de quelques %(w/w), de façon générale entre 0,4 et 3% (w/w) préférentiellement entre 0,5 et 1.5% (w/w)

Selon un mode de réalisation de l'invention, la poolish est obtenue par la maturation d'une composition comprenant de la farine, de l'eau, éventuellement du sucre et/ou des enzymes par au moins une levure choisie dans le groupe comprenant les levures de vinification, de brasserie et/ou de panification.
Selon l'invention, on entend par « poolish contenant de 20 à 55 g/L d'éthanol, de préférence de 35 à 50 g/L d'éthanol » une poolish qui produit de 20 à 55 g/L d'éthanol, de préférence de 35 à 50 g/L d'éthanol, lors de la maturation de la composition comprenant de la farine, de l'eau, éventuellement du sucre et/ou des enzymes, par la levure de vinification, de brasserie et/ou de panification.

La levure de vinification, de brasserie et/ou de panification utilisée pour la la maturation de la composition comprenant de la farine, de l'eau, éventuellement du sucre et/ou des enzymes, peut être appelée « levure de pré-fermentation ». De manière préférentielle, cette levure de pré-fermentation est une levure obtenue par culture de souches *Saccharomyces,* de préférence *Saccharomyces cerevisiae.* A titre d'exemple, on peut citer les produits commerciaux référencés suivants : Safale S04, Saflager W34/70, Safale US-05, Safbrew F2, Saflager S-189, Safbrew T58, Safoeno BC S103, Safale BE-134, Safale BE-256, Safale K97, Safoeno CK S102, Safbrew WB06, Safale S23, Florapan Lallemand, UCLM S377, Saf Instant Red, Saf Instant Gold, levure pressée hirondelle Bleue, levure pressée Hirondelle Or.

Selon un mode de réalisation de l'invention, la durée de l'étape de préparation de la poolish, c'est-à-dire la durée de maturation, peut-être analogue à la durée classique de l'étape de pré-fermentation d'un procédé sponge and dough, c'est-à-dire de 3 à 12 heures, mais elle peut être plus longue. Cette durée est généralement d'au moins 4 heures, voir d'au moins 8 heures et même d'au moins 12 heures. Cette durée peut aller jusqu'à 24 heures. A titre d'exemple on pourra citer une durée de 18 heures, et de préférence de 16 heures. Toujours à titre d'exemple, on pourra également citer une durée de 4 heures. Toutefois si la durée est trop courte (inférieure à 3 heures), l'effet de conservation peut ne pas être optimum. En revanche, pour des raisons économiques, il est évident qu'une durée au-delà de 24 heures n'est pas recommandée.

Selon un autre mode de réalisation de l'invention, le rapport poolish/farine totale est ajusté de manière à ne pas altérer les propriétés organoleptiques du pain.

Particulièrement, la quantité de farine de la poolish comprend de 20 à 65% de la farine totale, de préférence de 25 à 55%, plus préférentiellement 30% ou 50% de la farine totale. En dessous de 20% l'effet anti-moisissure pourrait s'avérer insuffisant.

Le procédé de préparation de l'invention comprend une étape d'application à la surface du produit cuit de boulangerie d'une composition comprenant un levain vivant et/ou une levure vivante.

Selon un mode de réalisation, la composition appliquée à la surface du produit cuit de boulangerie peut comprendre du levain vivant et de la levure vivante.
Selon encore un autre mode de réalisation, la composition appliquée à la surface du produit cuit de boulangerie peut comprendre du levain vivant ou de la levure vivante.

Selon l'invention, on entend par « composition comprenant un levain vivant » toute composition de levain liquide quel que soit son mode de préparation.

Selon un mode de réalisation de l'invention, le levain mis en œuvre est un levain sur farine de panification, par exemple sur farine de blé, farine de blé dur, farine de seigle, farine de seigle blanc ou farine de sarrasin.

Selon un autre mode de réalisation de l'invention, la composition de levain vivant liquide est appliquée à raison de 2 à 8 g, de préférence de 3 à 7 g et plus préférentiellement encore de 4 à 6 g de levain liquide présentant une matière sèche de 15 à 20% sur une surface totale de produit cuit de boulangerie de1200 à 1900 cm².

Dans la composition de levure vivante selon l'invention appliquée à la surface du produit cuit de boulangerie, la levure est la même que celle utilisé dans la poolish ou est différente. Il s'agit d'une levure de vinification, de brasserie et/ou de panification.

De manière préférentielle, cette levure est obtenue par culture de souches *Saccharomyces,* de préférence *Saccharomyces cerevisiae.* A titre d'exemple, on peut citer les produits commerciaux référencés suivants : Safale S04, Saflager W34/70, Safale US-05, Safbrew F2, Saflager S-189, Safbrew T58, Safoeno BC S103, Safale BE-134, Safale BE-256, Safale K97, Safoeno CK S102, Safbrew WB06, Safale S23, Florapan Lallemand, UCLM S377, Saf Instant Red, Saf Instant Gold, levure pressée hirondelle Bleue, levure pressée Hirondelle Or.

Selon encore un autre mode de réalisation de l'invention, la composition de levure vivante liquide est appliquée à raison de 2 à 8 g, de préférence de 3 à 7 g et plus préférentiellement encore de 4 à 6 g de composition liquide présentant une quantité de levure vivante de l'ordre de 10⁷ à 10¹⁰ CFU/L, de préférence de l'ordre de 10⁹ CFU/L sur une surface totale de produit cuit de boulangerie de1200 à 1900 cm².

Selon encore un mode de réalisation de l'invention, la composition est appliquée sur toute la surface du produit cuit de boulangerie. En particulier, dans le cas d'un produit cuit de boulangerie tranché, la composition est avantageusement appliquée sur toutes les surfaces externes des tranches.

La composition de levain vivant et/ou de levure liquide peut être appliquée par tout moyen connu tel que la pulvérisation le trempage. Le moyen préféré est la pulvérisation.

Le procédé selon l'invention est tout à fait approprié à la préparation de pain au levain, de pain de mie, de pain au lait, de pain brioché, de brioche, buns ou tout autre produit longue conservation.

En particulier, les produits cuits de boulangerie préparés selon le procédé de l'invention sont destinés à être emballés dans des emballages papier ou dans des emballages en film plastique de qualité alimentaire.

Selon un mode de réalisation particulier, le procédé de l'invention comprend en outre une étape d'emballage du produit cuit de boulangerie et une étape de pulvérisation de la composition de levain vivant et/ou de levure vivante dans ledit emballage avant sa fermeture.

Selon un autre aspect, il est proposé l'utilisation d'une poolish contenant de 20 à 55 g/L d'éthanol, de préférence de 35 à 50 g/L d'éthanol, conjointement avec l'application d'une composition de levain vivant et/ou de levure vivante à la surface d'un produit de boulangerie cuit, pour ralentir l'apparition de moisissures sur le produit cuit de boulangerie, notamment tranché.

Selon un mode de réalisation avantageux de l'invention, la farine de la poolish représente de 20 à 65%, de préférence au moins de 25 à 55% et notamment 30% ou 50% en poids de la farine totale du pain dans lequel la poolish est présente.

Selon un autre mode de réalisation, la poolish utilisée est une poolish d'au moins 4 heures de maturation, de préférence 8 heures de maturation, et plus préférentiellement encore d'au moins 12 heures de maturation.

Selon un autre mode de réalisation, la composition de levain vivant et/ou de levure vivante peut en outre être pulvérisée dans l'emballage fermé dans lequel il est conservé.

Les produits cuits de boulangerie obtenus selon le procédé de l'invention, de préférence tranchés, présentent une durée de conservation d'au moins 21 jours, de préférence d'au moins 28 jours, et ne comprennent pas d'additifs non naturels.

Les produits cuits de boulangerie obtenus selon le procédé de l'invention, de préférence tranchés, sont choisis parmi les pains au levain, les pains de mie, les pains briochés, les pains au lait, les brioches, buns ou tout autre produit longue conservation.

Les produits cuits de boulangerie obtenus selon le procédé de l'invention sont tranchés et emballés dans des emballages fermés qui sont des emballages papier ou des emballages en film plastique de qualité alimentaire.

L'invention va être décrite de façon plus détaillée à l'aide des exemples suivants qui sont donnés à titre d'illustration uniquement et des figures sur lesquelles :
**Fig. 1**

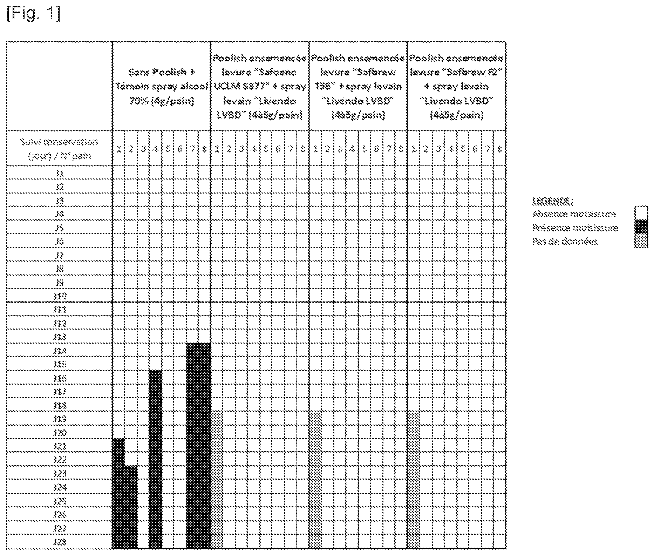

   [Fig. 1] est un graphique représentant la durée de conservation de pains préparés selon l'exemple 1.
**Fig. 2**

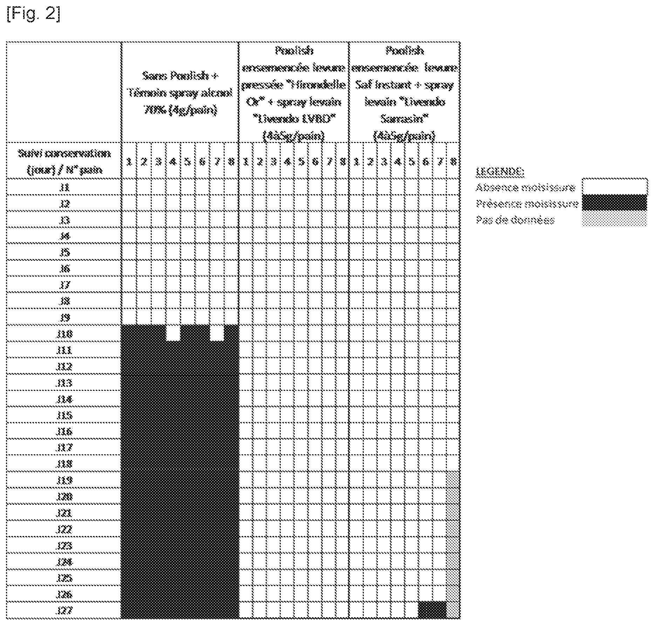

   [Fig. 2] est un graphique représentant la durée de conservation de pains préparés selon l'exemple 2.
**Fig. 3**

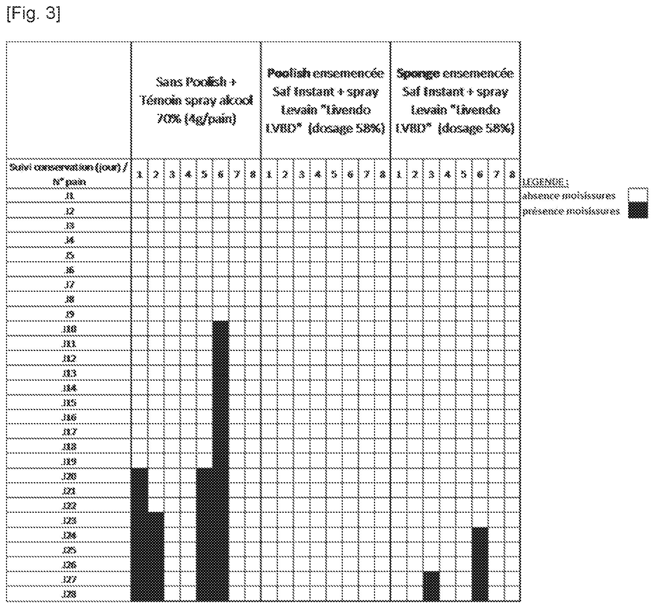

   [Fig. 3] est un graphique représentant la durée de conservation de pains préparés selon l'exemple 3.
**Fig. 4**

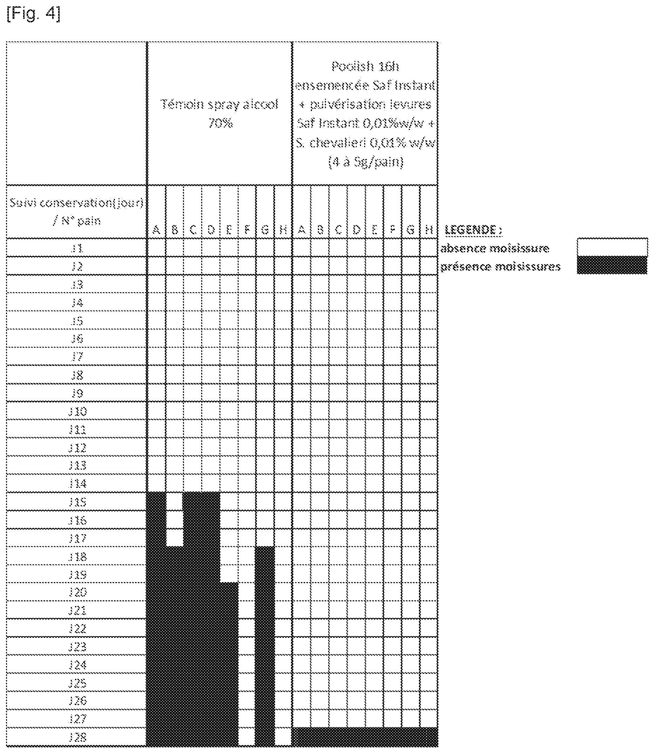

   [Fig. 4] est un graphique représentant la durée de conservation de pains préparés selon l'exemple 4.
**Fig. 5**

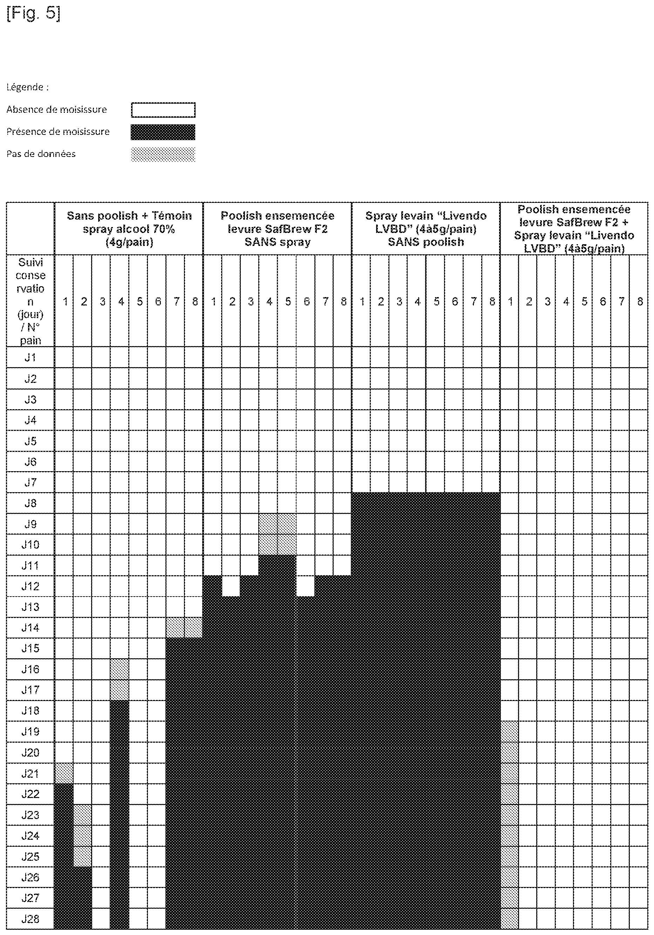

   [Fig.5] est un graphique représentant la durée de conservation de pains préparés selon l'exemple 5.

### Exemples

### Exemple 1 :

Le procédé de référence utilisé par la demanderesse est un procédé sponge and dough où l'étape du sponge et l'étape du dough ont les caractéristiques suivantes :

Avec ce procédé on a préparé 4 lots de pain de mie. Chaque lot comprend 8 pains de mie. Le premier lot est un lot de référence, sans poolish, dans lequel on a pulvérisé de l'alcool sur le pain de mie cuit à raison de 4 g/pain de mie cuit. Les 3 autres lots sont conformes à l'invention et utilisent chacun une levure différente dans la poolish, à savoir :
Lot 1 : Levure commercialisée par Lesaffre sous la dénomination Safoeno UCLM S377,
Lot 2 : Levure commercialisée par Lesaffre sous la dénomination Safbrew T58, Lot 3 : Levure commercialisée par Lesaffre sous la dénomination Safbrew F2.
Le pain mie cuit obtenu avec la poolish de chacun de ces lots est ensuite pulvérisé avec une composition de levain Livendo^{®} LVBD.

Dans la pâte, pour tous les lots, on a utilisé de la levure pressée « Hirondelle bleue^{®} » (désignée ci-après LP HB).
L'améliorant Ibis Violet^{®} est un améliorant de panification, commercialisé par Lesaffre, composé d'émulsifiants et permettant un meilleur volume du pain et une meilleure tenue de la pâte.
« Enz Pro 404 » désigne un mélange commercial d'alpha-amylase et amyloglucosidase.

Les composants mis en œuvre lors de chaque étape sont les suivants :

**[Tableau 1]**

| | Poolish | Pâte | Recette totale |
|---|---|---|---|
| Farine de tradition | 1100 | 2900 | 4000 |
| Eau | 1100 | 1100 | 2200 |
| Levure | 5,5 g levure (Safoeno UCLM S377 ou Safbrew T58 ou Safbrew F2) | 103,5 g LP HB | 5,5 g levure +103,5 g LP HB |
| Sel | 0 | 80 | 80 |
| Améliorant Ibis violet | 0 | 16 | 16 |
| Sucre | 110 | 200 | 310 |
| Beurre | 0 | 200 | 200 |
| Enz Pro 404 | 11 | 0 | 11 |

Les poolish sont fermentées pendant 16h à 30°C.

### Protocole de pulvérisation

La pulvérisation est réalisée avec l'outil de pulvérisation Dubor pour le levain Livendo^{®} LVBD (outil de micro-pulvérisation manuel TSA5 Dübor).
Précautions de stérilisation = stérilisation des grilles au four + nettoyage des mains à l'alcool avant manipulation des pains.
La pulvérisation s'effectue à une distance comprise entre 20 et 30 cm du produit sur toutes les faces du pain de mie.
La quantité totale de solution de levain pulvérisée visée est d'environ 4 à 5 g par pain.
Les pains sont non tranchés, pulvérisés et emballés individuellement après 90 min de ressuage (T=35°C à cœur).
Les pains sont stockés dans une armoire, dans le fournil, à température ambiante.

### Suivi de conservation

Evaluation visuelle du développement des moisissures sur les pains de mie. Notation quotidienne du nombre de pain avec/sans moisissure.
Les résultats sont présentés sur la figure 1.
Les pains sont séparés en 3 lots, chacun des lots est traité, emballé et conservé comme dans l'exemple 1.
La formation des moisissures est observée à l'œil nu chaque jour.

Les résultats sont donnés sur la figure 1. On observe sur la figure 1 que les pains avec poolish et traités par pulvérisation au levain ont une durée de vie plus longue que les pains sans poolish et traités par pulvérisation avec de l'alcool, et ceci quelle que soit la nature de la levure utilisée pour la poolish.

### Exemple 2 :

Le procédé de référence utilisé par la demanderesse est un procédé sponge and dough où l'étape du sponge et l'étape du dough ont les caractéristiques suivantes :

Avec ce procédé on a préparé 3 lots de pain de mie. Chaque lot comprend 8 pains de mie. Le premier lot est un lot de référence, sans poolish, dans lequel on a pulvérisé de l'alcool sur le pain de mie cuit à raison de 4 g/pain de mie cuit. Les 2 autres lots sont conformes à l'invention et utilisent chacun des levains différents lors de la pulvérisation sur le pain de cuit mie :
Lot 1 : Levain de blé dur Livendo^{®} LVBD (+ levure commercialisée par Lesaffre sous la dénomination Saf Instant dans la poolish),
Lot 2 : Levain de sarrasin Livendo^{®} LVSN (+ Levure commercialisée par Lesaffre sous la dénomination Saf Instant^{®} dans la poolish).
Dans la pâte, pour tous les lots, on a utilisé de la levure pressée « Hirondelle bleue^{®} » (désignée ci-après LP HB).

Les composants mis en œuvre lors de chaque étape sont les suivants :

**[Tableau 2]**

| | Poolish | Pâte | Recette totale |
|---|---|---|---|
| Farine de tradition | 1100 | 2900 | 4000 |
| Eau | 1100 | 1100 | 2200 |
| Levure | 5,5 g levure Saf Instant | 103,5 g LP HB | 5,5 g levure Saf instant +103,5 g LP HB |
| Sel | 0 | 80 | 80 |
| Améliorant Ibis violet | 0 | 16 | 16 |
| Sucre | 110 | 200 | 310 |
| Beurre | 0 | 200 | 200 |
| Enz Pro 404 | 11 | 0 | 11 |

Les poolish sont fermentées pendant 16h à 30°C.

### Protocole de pulvérisation

Pulvérisation réalisée avec l'outil de pulvérisation Dubor pour le levain Livendo^{®} LVBD et pour le levain Livendo^{®} LVSN (outil de micro-pulvérisation manuel TSA5 Dübor).
Précautions de stérilisation = stérilisation des grilles au four + nettoyage des mains à l'alcool avant manipulation des pains.
La pulvérisation s'effectue à une distance comprise entre 20 et 30 cm du produit sur toutes les faces du pain de mie.
La quantité totale de solution de levain pulvérisée visée est d'environ 4 à 5 g par pain.
Les pains sont non tranchés, pulvérisés et emballés individuellement après 90 min de ressuage (T=35°C à cœur).
Les pains sont stockés dans une armoire, dans le fournil, à température ambiante.

### Suivi de conservation

Evaluation visuelle du développement des moisissures sur les pains de mie. Notation quotidienne du nombre de pain avec/sans moisissure.

Les résultats de l'évaluation visuelle sont donnés sur la Figure 2. On observe sur la figure 2 que les pains avec poolish et traités par pulvérisation de levain ont une durée de vie plus longue que les pains sans poolish et traités par pulvérisation avec de l'alcool et ceci quelle que soit le levain vivant utilisé pour la pulvérisation.

### Exemple 3 :

Avec ce procédé on a préparé 3 lots de pain de mie. Chaque lot comprend 8 pains de mie. Le premier lot est un lot de référence sans poolish dans lequel on a pulvérisé de l'alcool sur le pain de mie cuit à raison de 4 g/pain de mie. Les 2 autres lots sont conformes à l'invention et utilisent des pré-fermentations de diverses hydratations (en fonction de l'hydratation, le levain levure préparé lors de l'étape de pré-fermentation sera appelé poolish ou sponge) :
Recette & process pour la préparation de la poolish :
   **Recette** :
      100% farine blé tradition,
      100% eau (32°C),
      1% Enz Pro 404,
      0,5% Levure SAF Instant^{®},
      10% glucose.
   **Process** :
      - 24h de maturation,
      - 30°C,
      - Pas d'agitation.
Recette & process pour la préparation du sponge :
   **Recette** :
      - 100% farine blé tradition,
      - 50% eau (32°C),
      - 1% Enz Pro 40,
      - 0,5% SAF Instant ^{®},
      - 10% glucose.
   **Process** :
      - 24h de maturation,
      - 30°C,
      - Pas d'agitation.
Le Tableau 3 ci-dessous présente la recette & le process du pain de mie sans poolish (témoin).

**[Tableau 3]**

| Ingrédients | poids | % |
|---|---|---|
| Farine de tradition | 4000 | 100 |
| Eau | 2200 | 55 |
| Levure LP HB | 103,5 | 3,0 |
| Sel | 80 | 1,8 |
| Améliorant Ibis violet | 16 | 0,5 |
| Sucre | 200 | 5,0 |
| Beurre | 200 | 5,0 |

**[Tableau 4]**

| DIAGRAMME de panification | |
|---|---|
| ETAPES | DOUGH |
| pétrissage | 4 + 6.5 min |
| pointage | 5 min |
| Division I | 720 g |
| boulage | oui |
| détente | 10 min |
| Façonnage | Machine/ twisté |
| Apprêt 1 80 grammes de pâte : 260 ml | 30°C / 85% d'humidité, 80 min |
| Cuisson | 25 min 220°C |

Le tableau 5 ci-dessous présente la recette & le process du pain de mie avec la poolish ou le songe.

**[Tableau 5]**

| Ingrédients | Poolish ou sponge | Pâte | Recette totale |
|---|---|---|---|
| Farine de tradition | 1100 | 2900 | 4000 |
| Eau | 1100 (poolish) ou 550 (sponge) | 1100 | 2200 |
| Levure | 5,5 g levure Saf Instant | 103,5 LP HB | 5,5 g levure Saf Instant +103,5 g LP HB |
| Sel | 0 | 80 | 80 |
| Améliorant Ibis violet | 0 | 16 | 16 |
| Sucre | 110 | 200 | 310 |
| Beurre | 0 | 200 | 200 |
| Enzymes Pro 404 | 11 | 0 | 11 |

### Protocole de pulvérisation

Pulvérisation manuelle réalisée avec des sprays manuels pour les levains Livendo LVBD.
Précautions de stérilisation = stérilisation des grilles au four + nettoyage des mains à l'alcool avant manipulation des pains.
La pulvérisation s'effectue à une distance comprise entre 20 et 30 cm du produit sur toutes les faces du pain de mie.
Le levain LVBD a été préalablement centrifugé afin d'éviter le bouchage de la buse du spray.
La quantité totale de solution de levain pulvérisée visée est d'environ 4 à 5 g avec le spray de jardinage.
Les pains sont non tranchés, pulvérisés et emballés individuellement après 90 min de ressuage (T=35°C à cœur).
Les pains sont stockés dans une armoire, dans le fournil, à température ambiante.

### Suivi de conservation

Evaluation visuelle du développement des moisissures sur les pains de mie. Notation quotidienne du nombre de pain avec/sans moisissure.
Remarque : les pains de mie industriels ont classiquement une date limite d'utilisation optimale (DLUO) de 21-28 jours.

L'observation visuelle a été faite et les résultats sont présentés sur la Figure 3. On observe sur la figure 3 que les pains avec poolish ou sponge et traités par pulvérisation de levain ont une durée de vie plus longue que les pains sans poolish et traités par pulvérisation avec de l'alcool, et ceci quelle que soit l'hydratation de la pré-fermentation (poolish ou sponge).

### Exemple 4 :

Le procédé de référence utilisé par la demanderesse est un procédé sponge and dough où l'étape du sponge et l'étape du dough ont les caractéristiques suivantes :

Avec ce procédé on a préparé 2 lots de pain de mie. Chaque lot comprend 8 pains de mie. Le premier lot est un lot de référence, sans poolish, dans lequel on a pulvérisé de l'alcool sur le pain de mie cuit à raison de 4 g/pain de mie cuit. L'autre lot est conforme à l'invention et utilise l'incorporation d'une poolish ensemencée par la levure Saf Instant et la pulvérisation sur produit cuit d'une solution de levures vivantes (mélange de levures Saf Instant et de *Saccharomyces chevalieri* chacune dosée à 0.01% w/w et diluées dans de l'eau à 30°C).

Dans la pâte, pour tous les lots, on a utilisé de la levure pressée « Hirondelle bleue^{®} » (désignée ci-après LP HB).

Les composants mis en œuvre lors de chaque étape sont les suivants :

**[Tableau 6]**

| | Poolish | Pâte | Recette totale |
|---|---|---|---|
| Farine de tradition | 1100 | 2900 | 4000 |
| Eau | 1100 | 1100 | 2200 |
| Levure | 5,5 g levure Saf Instant | 103,5 LP HB | 5,5 g levure Saf Instant + 103,5 g LP HB |
| Sel | 0 | 80 | 80 |
| Améliorant Ibis violet | 0 | 16 | 16 |
| Sucre | 110 | 200 | 310 |
| Beurre | 0 | 200 | 200 |
| Enzymes | 11 | 0 | 11 |

Les poolish sont fermentées pendant 16h à 30°C

### Protocole de pulvérisation

Pulvérisation réalisée avec l'outil de pulvérisation Dubor pour la solution de levures vivantes (outil de micro-pulvérisation manuel TSA5 Dübor).
Précautions de stérilisation = stérilisation des grilles au four + nettoyage des mains à l'alcool avant manipulation des pains.
La pulvérisation s'effectue à une distance comprise entre 20 et 30 cm du produit sur toutes les faces du pain de mie.
La quantité totale de solution de levures pulvérisée visée est d'environ 4 à 5 g par pain.
Les pains sont non tranchés, pulvérisés et emballés individuellement après 90 min de ressuage (T=35°C à cœur).
Les pains sont stockés dans une armoire, dans le fournil, à température ambiante.

### Suivi de conservation

Evaluation visuelle du développement des moisissures sur les pains de mie. Notation quotidienne du nombre de pain avec/sans moisissure.
Les résultats sont présentés sur la figure 4.
La formation des moisissures est observée à l'œil nu chaque jour.

### Les résultats sont donnés sur la figure 4.

On observe sur la figure 4 que les pains avec poolish et traités par pulvérisation avec une solution de levures ont une durée de vie plus longue que les pains sans poolish et traités par pulvérisation avec de l'alcool.

### Exemple 5

Cet exemple est une illustration de l'effet synergique entre la poolish et le sprayage à la surface du produit cuit de boulangerie d'une solution comprenant un levain vivant.

Le procédé de référence utilisé par la demanderesse est un procédé sponge and dough où l'étape du sponge et l'étape du dough ont les caractéristiques suivantes :

Avec ce procédé on a préparé 4 lots de pain de mie. Chaque lot comprend 8 pains de mie. Le premier lot est un lot de référence, sans poolish, dans lequel on a pulvérisé de l'alcool sur le pain de mie cuit à raison de 4 g/pain de mie cuit. Dans le deuxième lot, les pains de mie sont fabriqués selon le procédé de sponge and dough à partir de levure commercialisée par Lesaffre sous la dénomination Safbrew F2 mais sans aucune pulvérisation après cuisson. Dans le troisième lot, les pains de mies ne contiennent pas de poolish mais sont pulvérisés avec une composition de levain Livendo^{®} LVBD après cuisson. Le quatrième lot est conforme à l'invention, la poolish est fabriquée à partir de levure commercialisée par Lesaffre sous la dénomination Safbrew F2 puis le pain de mie cuit obtenu avec la poolish est ensuite pulvérisé avec une composition de levain Livendo^{®} LVBD.

Dans la pâte, pour tous les lots, on a utilisé de la levure pressée « Hirondelle bleue^{®} » (désignée ci-après LP HB).

Les composants mis en œuvre lors de chaque étape sont les suivants :

**[Tableau 7]**

| | Poolish | Pâte | Recette totale |
|---|---|---|---|
| Farine de tradition | 1100 | 2900 | 4000 |
| Eau | 1100 | 1100 | 2200 |
| Levure | 5,5 g levure Safbrew F2 | 103,5 LP HB | 5,5 g levure +103,5 g LP HB |
| Sel | 0 | 80 | 80 |
| Améliorant Ibis violet | 0 | 16 | 16 |
| Sucre | 110 | 200 | 310 |
| Beurre | 0 | 200 | 200 |
| Enzymes | 11 | 0 | 11 |

Les poolish sont fermentées pendant 24h à 30°C.

**[Tableau 8]**

| | Pâte |
|---|---|
| Farine de tradition | 4000 |
| Eau | 2200 |
| Levure | 103,5 g LP HB |
| Sel | 80 |
| Améliorant Ibis violet | 16 |
| Sucre | 200 |
| Beurre | 200 |
| Enz Pro 404 | 11 |

### Protocole de pulvérisation

La pulvérisation est réalisée avec l'outil de pulvérisation Dubor pour le levain Livendo^{®} LVBD (outil de micro-pulvérisation manuel TSA5 Dübor).
Précautions de stérilisation = stérilisation des grilles au four + nettoyage des mains à l'alcool avant manipulation des pains.
La pulvérisation s'effectue à une distance comprise entre 20 et 30 cm du produit sur toutes les faces du pain de mie.
La quantité totale de solution de levain pulvérisée visée est d'environ 4 à 5 g par pain.
Les pains sont non tranchés, pulvérisés et emballés individuellement après 90 min de ressuage (T=35°C à cœur).
Les pains sont stockés dans une armoire, dans le fournil, à température ambiante.

### Suivi de conservation

Evaluation visuelle du développement des moisissures sur les pains de mie. La formation des moisissures est observée à l'œil nu chaque jour Notation quotidienne du nombre de pain avec/sans moisissure.
Remarque : les pains de mie industriels ont classiquement une DLUO de 21-28 jours.

Les résultats de l'évaluation visuelle sont donnés sur la Figure 5. On observe sur la figure 5 que les pains de mie avec poolish et sans pulvérisation au levain et les pains de mie sans poolish mais pulvérisés au levain subissent plus tôt l'apparition de moisissures que les pains de mies avec poolish et pulvérisés au levain. Ceci témoigne d'un effet synergique entre la poolish et la pulvérisation au levain. C'est l'action combinée des deux éléments (poolish + pulvérisation) qui permet l'obtention d'un effet anti-moisissures augmenté en utilisant uniquement des produits naturels.

## Revendications

1. Procédé de préparation de produit cuit de boulangerie résistant aux moisissures comprenant la cuisson d'une pâte obtenue par une méthode de panification de type sponge and dough mettant en œuvre une poolish contenant de 20 à 55 g/L d'éthanol, de préférence de 35 à 50 g/L d'éthanol, et comprenant après la cuisson une étape d'application à la surface du produit cuit de boulangerie d'une solution comprenant un levain vivant et/ou une levure vivante.

2. Procédé selon la revendication 1 **caractérisé en ce que** ladite poolish est obtenue par la maturation d'une composition comprenant de la farine, de l'eau, éventuellement du sucre et/ou des enzymes par au moins une levure choisie dans le groupe comprenant les levures de vinification, de brasserie et/ou de boulangerie.

3. Procédé selon l'une des revendications précédentes, **caractérisé en ce que** la levure est obtenue par culture de souche Saccharomyces cerevisiae.

4. Procédé selon l'une des revendications précédentes, **caractérisé par le fait que** la durée de maturation de la poolish est d'au moins 4 heures, de préférence d'au moins 8 heures et plus préférentiellement encore d'au moins 12 heures.

5. Procédé selon l'une des revendications précédentes, **caractérisé en ce que** la farine de la poolish représente 20 à 65% de la farine totale, de préférence 25 à 55%, plus préférentiellement encore 30% ou 50% de la farine totale.

6. Procédé selon l'une des revendications précédentes, dans lequel le levain vivant appliqué à la surface du produit cuit de boulangerie est un levain sur toute farine de panification, de préférence sur farine de blé, farine de blé dur, farine de seigle, farine de seigle blanc ou farine de sarrasin.

7. Procédé selon l'une des revendications précédentes, dans lequel la composition de levain vivant est appliquée à raison de 2 à 8 g, de préférence de 3 à 7 g et plus préférentiellement encore de 4 à 6 g de levain liquide présentant une matière sèche de 15 à 20% sur une surface de produit cuit de boulangerie de 1200 à 1900 cm².

8. Procédé selon l'une des revendications 1 à 7, dans lequel la composition de levure vivante est appliquée à raison de 2 à 8 g, de préférence de 3 à 7 g et plus préférentiellement encore de 4 à 6 g de composition liquide présentant une quantité de levures vivantes de l'ordre de 10⁷ à 10¹⁰ CFU/L, de préférence de l'ordre de 10⁹ CFU/L sur une surface de produit cuit de boulangerie de 1200 à 1900 cm².

9. Procédé selon l'une des revendications précédentes, dans lequel la composition de levain vivant est appliquée par trempage ou pulvérisation.

10. Procédé selon l'une des revendications précédentes, pour la préparation de pain au levain, de pain de mie, de pain au lait, de pain brioché, de brioche, buns ou tout autre produit longue conservation

11. Procédé selon l'une des revendications précédentes pour la préparation de produits cuits de boulangerie, notamment tranchés, destinés être emballés dans des emballages papier ou dans des emballages en film plastique de qualité alimentaire.

12. Utilisation d'une poolish contenant de 20 à 55 g/L d'éthanol, de préférence de 35 à 50 g/L d'éthanol, conjointement avec l'application d'une composition de levain vivant et/ou de levure vivante à la surface d'un produit de boulangerie cuit, pour ralentir l'apparition de moisissures sur le produit cuit de boulangerie, notamment tranché.

13. Utilisation selon la revendication 12, **caractérisée par le fait que** la farine de la poolish représente 20 à 65%, de préférence 25 à 55% et notamment 30% ou 50% en poids de la farine totale du pain dans lequel la poolish est présente.

14. Utilisation selon la revendication 12 ou 13, dans laquelle la composition de levain vivant et/ou de levure vivante peut en outre être pulvérisée dans l'emballage fermé dans lequel il est conservé.

## Patentansprüche

1. Verfahren zur Herstellung einer schimmelresistenten gebackenen Backware, umfassend das Backen eines Teigs, der durch ein Sponge-and-Dough-Brotbackverfahren unter Verwendung eines Poolish-Teigs mit einem Ethanolgehalt von 20 bis 55 g/l, vorzugsweise von 35 bis 50 g/l Ethanol, erhalten wird, und nach dem Backen einen Schritt des Aufbringens einer Lösung, die einen lebenden Sauerteig und/oder eine lebende Hefe umfasst, auf die Oberfläche des Backprodukts.

2. Verfahren nach Anspruch 1, **dadurch gekennzeichnet, dass** der Poolish durch Reifung einer Zusammensetzung erhalten wird, die Mehl, Wasser, ggf. Zucker und/oder Enzyme umfasst, durch mindestens eine Hefe, die aus der Gruppe ausgewählt ist, die Wein-, Brau- und/oder Backhefen umfasst.

3. Verfahren nach einem der vorhergehenden Ansprüche, **dadurch gekennzeichnet, dass** die Hefe durch Kultivierung des Stammes Saccharomyces cerevisiae erhalten wird.

4. Verfahren nach einem der vorhergehenden Ansprüche, **dadurch gekennzeichnet, dass** die Reifungsdauer des Poolish mindestens 4 Stunden, vorzugsweise mindestens 8 Stunden und noch bevorzugter mindestens 12 Stunden beträgt.

5. Verfahren nach einem der vorhergehenden Ansprüche, **dadurch gekennzeichnet, dass** das Mehl des Poolish 20 bis 65 % des Gesamtmehls, vorzugsweise 25 bis 55 %, noch bevorzugter 30 % oder 50 % des Gesamtmehls ausmacht.

6. Verfahren nach einem der vorhergehenden Ansprüche, wobei der auf die Oberfläche der gebackenen Backware aufgetragene lebende Sauerteig ein Sauerteig auf Basis von jeglichem Mehl für die Brotherstellung, vorzugsweise auf Weizenmehl, Hartweizenmehl, Roggenmehl, weißem Roggenmehl oder Buchweizenmehl ist.

7. Verfahren nach einem der vorhergehenden Ansprüche, wobei die lebende Sauerteigzusammensetzung in einer Menge von 2 bis 8 g, vorzugsweise 3 bis 7 g und noch bevorzugter 4 bis 6 g flüssigem Sauerteig mit einem Trockensubstanzgehalt von 15 bis 20 % auf eine Oberfläche der gebackenen Backware von 1200 bis 1900 cm² aufgetragen wird.

8. Verfahren nach einem der Ansprüche 1 bis 7, wobei die lebende Hefezusammensetzung in einer Menge von 2 bis 8 g, vorzugsweise 3 bis 7 g und noch bevorzugter 4 bis 6 g flüssiger Zusammensetzung, mit einem Gehalt an lebenden Hefen in der Größenordnung von 10⁷ bis 10¹⁰ CFU/L, vorzugsweise in der Größenordnung von 10⁹ CFU/L, auf eine Oberfläche der gebackenen Backware von 1200 bis 1900 cm² aufgetragen wird.

9. Verfahren nach einem der vorhergehenden Ansprüche, wobei die lebende Hefezusammensetzung durch Eintauchen oder Besprühen aufgebracht wird.

10. Verfahren nach einem der vorhergehenden Ansprüche zur Herstellung von Sauerteigbrot, Toastbrot, Milchbrot, Briochebrot, Brioche, Brötchen oder jeglichen anderen haltbaren Backwaren

11. Verfahren nach einem der vorhergehenden Ansprüche zur Herstellung von gebackenen Backwaren, insbesondere geschnittenen Backwaren, die in Papierverpackungen oder in lebensmittelechten Kunststofffolienverpackungen verpackt werden sollen.

12. Verwendung eines Poolish, der 20 bis 55 g/l Ethanol, vorzugsweise 35 bis 50 g/l Ethanol enthält, in Verbindung mit dem Auftragen einer lebenden Sauerteigzusammensetzung und/oder einer lebenden Hefezusammensetzung auf die Oberfläche einer gebackenen Backware, um das Auftreten von Schimmel auf den gebackenen Backwaren, insbesondere geschnittenen Backwaren, zu verlangsamen.

13. Verwendung nach Anspruch 12, **dadurch gekennzeichnet, dass** das Mehl des Poolish 20 bis 65 Gew.-%, vorzugsweise 25 bis 55 Gew.-% und insbesondere 30 Gew.-% oder 50 Gew.-% des Gesamtmehls des Brotes ausmacht, in dem der Polish enthalten ist.

14. Verwendung nach Anspruch 12 oder 13, wobei die lebende Sauerteigzusammensetzung und/oder die lebende Hefezusammensetzung außerdem in der geschlossenen Verpackung, in der sie aufbewahrt wird, pulverisiert sein kann.

## Claims

1. A method for preparing mold-resistant cooked bakery products comprising cooking a dough obtained by a breadmaking method of the sponge and dough type using a poolish containing from 20 to 55 g/L of ethanol, preferably from 35 to 50 g/L of ethanol, and comprising, after cooking, a step of application of a solution comprising a live leaven and/or a live yeast on the surface of the cooked bakery product.

2. The method as claimed in claim 1, **characterized in that** said poolish is obtained by the maturation of a composition comprising flour, water, optionally sugar and/or enzymes by at least one yeast selected from the group comprising wine yeast, brewer's yeast and/or baker's yeast.

3. The method as claimed in one of the preceding claims, **characterized in that** yeast is obtained by culture of the strain Saccharomyces cerevisiae.

4. The method as claimed in one of the preceding claims, **characterized in that** the maturation time of the poolish is at least 4 hours, preferably at least 8 hours and even more preferably at least 12 hours.

5. The method as claimed in one of the preceding claims, **characterized in that** the flour of the poolish represents 20 to 65% of the total flour, preferably 25 to 55%, even more preferably 30% or 50% of the total flour.

6. The method as claimed in one of the preceding claims, in which the live leaven applied on the surface of the cooked bakery product is a leaven for any baking flour, preferably for wheat flour, hard wheat flour, rye flour, white rye flour or buckwheat flour.

7. The method as claimed in one of the preceding claims, in which the composition of live leaven is applied at a rate from 2 to 8 g, preferably from 3 to 7 g and even more preferably from 4 to 6 g of liquid leaven having dry matter from 15 to 20% on a surface area of cooked bakery product from 1200 to 1900 cm².

8. The method as claimed in one of claims 1 to 7, in which the composition of live yeast is applied at a rate from 2 to 8 g, preferably from 3 to 7 g and even more preferably from 4 to 6 g of liquid composition having a quantity of live yeasts of the order of 10⁷ to 10¹⁰ CFU/L, preferably of the order of 10⁹ CFU/L on a surface area of cooked bakery product from 1200 to 1900 cm².

9. The method as claimed in one of the preceding claims, in which the composition of live leaven is applied by dipping or spraying.

10. The method as claimed in one of the preceding claims, for preparing sourdough bread, sandwich bread, milkbread, milk loaf, brioche, buns or any other long-life product

11. The method as claimed in one of the preceding claims for preparing cooked bakery products, in particular sliced, intended to be packaged in paper packaging or in packaging made of food-grade plastic film.

12. A use of a poolish containing from 20 to 55 g/L of ethanol, preferably from 35 to 50 g/L of ethanol, jointly with the application of a composition of live leaven and/or of live yeast on the surface of a cooked bakery product, to slow down the appearance of molds on cooked bakery products, in particular if sliced.

13. The use as claimed in claim 12, **characterized in that** the flour of the poolish represents 20 to 65%, preferably 25 to 55% and in particular 30% or 50 wt% of the total flour of the bread in which the poolish is present.

14. The use as claimed in claim 12 or 13, in which the composition of live leaven and/or of live yeast may additionally be sprayed in the closed packaging in which it is stored.
